(19) Europäisches Patentamt
European Patent Office
Office européen des brevets

(11) Veröffentlichungsnummer: **0 559 044 A1**

# (12) EUROPÄISCHE PATENTANMELDUNG

(21) Anmeldenummer: **93102758.5**

(22) Anmeldetag: **22.02.93**

(51) Int. Cl.[5]: **C07D 251/46**, A01N 47/36, C07D 251/16, C07D 239/52

(30) Priorität: **05.03.92 DE 4206918**
**19.11.92 DE 4239002**

(43) Veröffentlichungstag der Anmeldung:
**08.09.93 Patentblatt 93/36**

(84) Benannte Vertragsstaaten:
**BE CH DE DK FR GB IT LI NL SE**

(71) Anmelder: **BAYER AG**
**D-51368 Leverkusen(DE)**

(72) Erfinder: **Müller, Klaus-Helmut, Dr.**
**Bockhackstrasse 55**
**W-4000 Düsseldorf 13(DE)**

## (54) Verfahren zur Herstellung von Sulfonylharnstoff-Salzen.

(57) Die Erfindung betrifft ein neues Verfahren zur Herstellung von herbizid wirksamen Sulfonylharnstoff-Salzen der Formel (I)

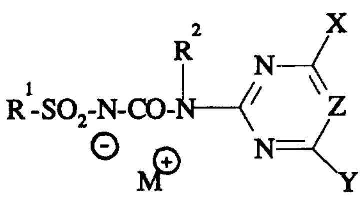

(I)

in welcher
- $M^+$ für ein Alkalimetallion oder ein Erdalkalimetallionenäquivalent steht,
- Z für N, CH oder C-Halogen steht,
- $R^1$ für jeweils gegebenenfalls substituiertes Aryl, Aralkyl oder Heteroaryl steht,

(wobei $R^2$,X und Y die in der Beschreibung angegebenen Bedeutungen haben),
welches dadurch gekennzeichnet ist, daß man zunächst Sulfonsäureamide der allgemeinen Formel (II)

$R^1$-$SO_2$-$NH_2$ (II)

mit Alkalimetall-hydroxiden oder -alkoholaten oder mit Erdalkalimetall-hydroxidenoder -alkoholaten gegebenenfalls in Gegenwart eines Verdünnungsmittels bei Temperaturen zwischen -20 °C und +150 °C umsetzt (1. Stufe) und dann die hierbei gebildeten Sulfonamid-Salze der allgemeinen Formel (IIa)

$R^1$-$SO_2$-NH-$M^+$ (IIa)

mit Urethanen (Carbamaten) der allgemeinen Formel (III)

EP 0 559 044 A1

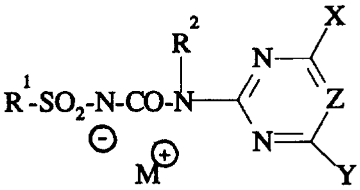

(I)

(in welcher $R^3$ für Alkyl, Aralkyl oder Aryl steht),
gegebenenfalls in Gegenwart eines Verdünnungsmittels bei Temperaturen zwischen -20°C und +150°C umsetzt (2. Stufe) und die Produkte der Formel (I) nach üblichen Methoden isoliert.

Die Erfindung betrifft ein neues Verfahren zur Herstellung von Sulfonylharnstoff-Salzen, welche als Herbizide verwendet werden können.

Es ist bekannt, daß man Sulfonylharnstoff-Salze erhält, wenn man Lösungen von Sulfonylharnstoffen in Halogenkohlenwasserstoff-Solventien mit Alkalimetall- oder Erdalkalimetall-hydroxiden umsetzt und, gegebenenfalls nach Filtration, das Lösungsmittel abdestilliert (vgl. EP-A 304 282).

Da bei dieser Verfahrensweise im allgemeinen keine Kristallisation des Produktes aus der Lösung erfolgt, ist eine energieaufwendige Destillation des Lösungsmittels erforderlich und die Chance, einen Reinigungseffekt zu erzielen, kaum gegeben.

Weiter ist bekannt, daß Sulfonylharnstoff-Salze durch Umsetzung von Sulfonylharnstoffen mit Alkalimetallhydroxiden oder mit basischen organischen Stickstoffverbindungen in Gegenwart von Kohlenwasserstoffen als Verdünnungsmitteln in guten Ausbeuten und in hoher Reinheit erhalten werden können (vgl. EP-A 433779).

Es wurde nun gefunden, daß man Sulfonylharnstoff-Salze der allgemeinen Formel (I)

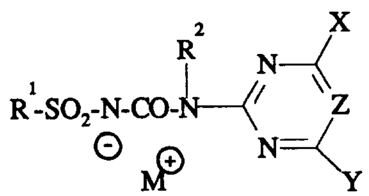

(I)

in welcher

$M^+$ für ein Alkalimetallion oder ein Erdalkalimetallionenäquivalent steht,

$R^1$ für jeweils gegebenenfalls substituiertes Aryl, Aralkyl oder Heteroaryl steht,

$R^2$ für Wasserstoff oder für jeweils gegebenenfalls substituiertes Alkyl, Alkenyl, Alkinyl oder Aralkyl steht,

X für Wasserstoff, Halogen oder für jeweils gegebenenfalls durch Halogen oder Alkoxy substituiertes Alkyl, Alkoxy, Alkylthio oder Alkylamino steht,

Y für Wasserstoff, Halogen oder für jeweils gegebenenfalls durch Halogen substituiertes Alkyl oder Alkoxy steht und

Z für Stickstoff, eine CH-Gruppierung oder eine C-Halogen-Gruppierung steht,

in sehr guten Ausbeuten und in hoher Reinheit erhält, wenn man zunächst Sulfonsäureamide der allgemeinen Formel (II)

$R^1$-$SO_2$-$NH_2$ (II)

in welcher

$R^1$ die oben angegebene Bedeutung hat,

mit Alkalimetall-hydroxiden oder -alkoholaten oder mit Erdalkalimetall-hydroxidenoder -alkoholaten gegebenenfalls in Gegenwart eines Verdünnungsmittels bei Temperaturen zwischen -20 °C und +150 °C umsetzt (1. Stufe)

und dann die hierbei gebildeten Sulfonamid-Salze der allgemeinen Formel (IIa)

$R^1$-$SO_2$-$NH$-$M^+$ (IIa)

in welcher

M und $R^1$ die oben angegebene Bedeutung haben,

mit Urethanen (Carbarnaten) der allgemeinen Formel (III)

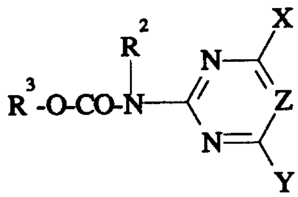

(III)

in welcher

$R^2$, X, Y und Z die oben angegebene Bedeutung haben und

$R^3$ für Alkyl, Aralkyl oder Aryl steht,

gegebenenfalls in Gegenwart eines Verdünnungsmittels bei Temperaturen zwischen -20 °C und +150 °C umsetzt (2. Stufe) und die Produkte der Formel (I) nach üblichen Methoden isoliert.

Es ist als überraschend anzusehen, daß die Herstellung von Sulfonylharnstoff-Salzen ohne Zwischervsolierung der entsprechenden freien Sulfonylharnstoffe in sehr guten Ausbeuten und in hoher Reinheit ausgehend von den Sulfonsäureamiden der Formel (II) und den Urethanen der Formel (III) gelingt, wobei die Vorbehalte bezüglich der verwendbaren Verdünnungsmittel überwunden werden (vgl. EP-A 433779).

Das erfindungsgemäße Verfahren betrifft vorzugsweise die Herstellung von Sulfonylharnstoff-Salzen der Formel (I), in welcher

$M^+$ für ein Lithium-, Natrium- oder Kaliumion oder für ein Magnesium- oder Calciumionenäquivalent steht,

$R^1$ für jeweils gegebenenfalls substituiertes Phenyl, Naphthyl, Benzyl, Pyridyl, Thienyl oder Pyrazolyl steht, wobei die möglichen Substituenten vorzugsweise ausgewählt sind aus der Reihe Halogen, Carboxy, Cyano, Carbamoyl, Nitro, Amino, $C_1$-$C_4$-Alkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkoxy-$C_1$-$C_4$-alkoxy, $C_1$-$C_4$-Alkylthio, $C_1$-$C_4$-Alkylsulfinyl, $C_1$-$C_4$-Alkylsulfonyl, Di-($C_1$-$C_4$-alkyl)-amino-sulfonyl, N-$C_1$-$C_4$-Alkoxy-N-$C_1$-$C_4$-alkyl-amino-sulfonyl, Phenyl, Phenoxy, $C_1$-$C_4$-Alkoxy-carbonyl, $C_1$-$C_4$-Halogenalkoxy-carbonyl, $C_1$-$C_2$-Alkoxy-$C_1$-$C_2$-alkoxy-carbonyl, Di-($C_1$-$C_4$-alkyl)-amino-carbonyl,

$R^2$ für Wasserstoff, für $C_1$-$C_6$-Alkyl (welches gegebenenfalls durch Fluor, Cyano, $C_1$-$C_4$-Alkoxy oder $C_1$-$C_4$-Alkylthio substituiert ist), für $C_3$-$C_6$-Alkenyl und $C_3$-$C_6$-Alkinyl (welche gegebenenfalls durch Fluor oder Chlor substituiert sind) oder für Phenyl-$C_1$-$C_2$-alkyl (welches im Phenylteil gegebenenfalls durch Fluor, Chlor, Nitro, Cyano, Methyl, Methoxy oder $C_1$-$C_2$-Alkoxycarbonyl substituiert ist) steht,

X für Wasserstoff, Halogen oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio oder $C_1$-$C_4$-Alkylamino steht,

Y für Wasserstoff, Halogen oder für jeweils gegebenenfalls durch Halogen substituiertes $C_1$-$C_4$-Alkyl oder $C_1$-$C_4$-Alkoxy steht und

Z für Stickstoff oder eine CH-Gruppierung steht.

Das erfindungsgemäße Verfahren betrifft insbesondere die Herstellung von Sulfonylharnstoff-Salzen der Formel (I), in welcher

$M^+$ für ein Natriumion oder ein Kaliumion steht,

$R^1$ für jeweils in ortho-Position durch Fluor, Chlor,Brom, Methyl, Ethyl, Trifluormethyl, Methoxy, Ethoxy, Difluormethoxy, Trifluormethoxy, Chlorethoxy, Methoxyethoxy, Ethoxyethoxy, Methylthio, Ethylthio, Propylthio, Methylsulfinyl, Ethylsulfinyl, Propylsulfinyl, Methylsulfonyl, Ethylsulfonyl, Propylsulfonyl, Dimethylaminosulfonyl, Diethylaminosulfonyl, N-Methoxy-N-methyl-amino-sulfonyl, Phenyl, Methoxycarbonyl, Ethoxycarbonyl, Propoxycarbonyl, Isopropoxycarbonyl, Chlorethoxycarbonyl, Methoxyethoxycarbonyl, Dimethylarninocarbonyl oder Diethylaminocarbonyl und gegebenenfalls in einer weiteren Position durch Halogen substituiertes Phenyl oder Benzyl, für 3-Dimethylaminocarbonyl-pyridin-2-yl oder 3-Ethylsulfonyl-pyridin-2-yl, für 2-Methoxycarbonyl-thiophen-3-yl oder für 1-Methyl-4-methoxycarbonyl-pyrazol-5-yl, 1-Methyl-4-ethoxycarbonyl-pyrazol-5-yl, 1-Methyl-3-chlor-4-methoxycarbonyl-pyrazol-5-yl, 1-Methyl-3-chlor-4-ethoxycarbonyl-pyrazol-5-yl oder 1-Pyridyl-4-methoxycarbonyl-pyrazol-5-yl steht,

$R^2$ für Wasserstoff oder Methyl steht,

X für Wasserstoff, Chlor, Methyl, Ethyl, Trichlormethyl, Trifluormethyl, Methoxymethyl, Methoxy, Ethoxy, Difluormethoxy, Fluorethoxy, Difluorethoxy, Trifluorethoxy, Methoxyethoxy, Methylthio, Ethylthio, Methylamino oder Ethylamino steht,

Y für Wasserstoff, Chlor, Methyl, Ethyl, Trifluormethyl, Methoxy, Ethoxy oder Difluormethoxy steht,

und

Z für Stickstoff oder eine CH-Gruppierung steht.

Verwendet man beispielsweise 2-Difluormethoxy-benzolsulfonsäureamid und Natrium-methylat als Ausgangsstoffe und setzt das damit gebildete Natriunisalz mit N-Methyl-N-(4,6-dimethoxy-1,3,5-triazin-2-yl)-carbamidsäure-phenylester um, so kann der Reaktionsablauf beim ertindungsgemäßen Verfahren durch das folgende Formelschema skizziert werden:

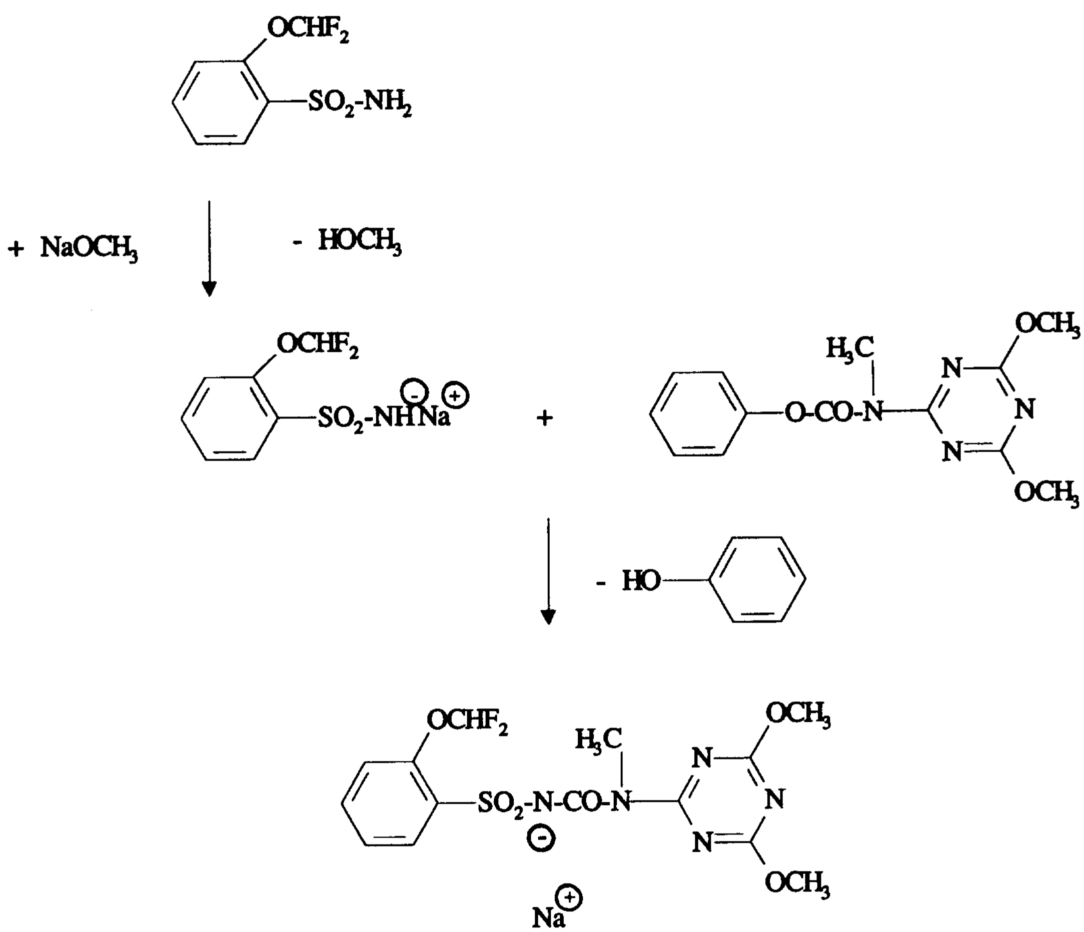


Die beim erfindungsgemäßen Verfahren als Ausgangsstoffe zu verwendenden Sulfonsäureamide sind durch die Formel (II) allgemein definiert. In Formel (II) hat $R^1$ vorzugsweise bzw. insbesondere diejenige Bedeutung, die bereits oben im Zusammenhang mit der Beschreibung der erfindungsgemäß herzustellenden Verbindungen der Formel (I) vorzugsweise bzw. als insbesondere bevorzugt für $R^1$ angegeben wurde.

Als Beispiele für die Ausgangsstoffe der Formel (II) seien genannt:

2-Fluor-, 2-Chlor-, 2-Brom-, 2,5-Dichlor-, 2,6-Dichlor-, 2-Nitro-, 2-Cyano-, 2-Methyl-, 2-Trifluormethyl-, 2-Methoxy-, 2-Ethoxy-, 2-Difluormethoxy-, 2-Trifluormethoxy-, 2-Methylthio-, 2-Ethylthio-, 2-(2-Chlor-ethoxy)-, 2-(2-Methoxy-ethoxy)-, 2-Dimethylaminosulfonyl-, 2-Phenyl-, 2-Methoxycarbonyl-, 2-Ethoxycarbonyl- und 2-Propoxycarbonyl-benzolsulfonsäureamid, (2-Chlor-phenyl)-, (2-Difluormethoxy-phenyl)-, (2-Trifluormethoxy-phenyl)-, (2-Methoxycarbonyl-phenyl)- und (2-Ethoxycarbonyl-phenyl)-methansulfonsäureamid, 3-Dimethylaminocarbonyl-pyridin-2-sulfonsäureamid, 3-Ethylsulfonyl-pyridin-2-sulfonsäureamid, 2-Methoxycarbonyl-thiophen-3-sulfonsäureamid, 1-Methyl-4-methoxycarbonyl-, 1-Methyl-4-ethoxycarbonyl-, 1-Methyl-3-chlor-4-methoxycarbonyl-, 1-Methyl-3-chlor-4-ethoxycarbonyl- und 1-Pyridyl-4-methoxycarbonyl-pyrazol-5-sulfonsäureamid.

Die Suffonsäureamide der Formel (II) sind bekannt und/ oder können nach an sich bekannten Verfahren hergestellt werden (vgl. J. Org. Chem. 25 (1960), 1824; loc. cit. 33 (1968), 2104; DE-AS 2308262; EP-A 23140; EP-A 23141; EP-A 23422; EP-A 35893; EP-A 48143; EP-A 51466; EP-A 64322; EP-A 70041; EP-A 44808; EP-A 44809; US-P 2929820; US-P 4282242; US-P 4348220; US-P 4372778; US-P 4806147).

Die beim erfindungsgemäßen Verfahren zur Herstellung von Verbindungen der Formel (I) weiter als Ausgangsstoffe zu verwendenden Urethane sind durch die Formel (III) allgemein definiert.

In Formel (III) haben $R^2$, X, Y und Z vorzugsweise bzw. insbesondere diejenigen Bedeutungen, die bereits oben im Zusammenhang mit der Beschreibung der erfindungsgemäß herstellbaren Verbindungen der Formel (1) vorzugsweise bzw. als insbesondere bevorzugt für $R^2$, X, Y und Z angegeben wurden;

$R^3$ steht vorzugsweise für Methyl, Ethyl, Benzyl oder Phenyl, insbesondere für Phenyl.

Als Beispiele für die Ausgangsstoffe der Formel (III) seien genannt:

N-(4,6-Dimethyl-pyrimidin-2-yl)-, N-(4-Methoxy-6-methyl-pyrimidin-2-yl)-, N-(4,6-Dimethoxy-pyrimidin-2-yl)-, N-(4,6-Dimethyl-s-triazin-2-yl)-, N-(4-Methoxy-6-methyl-s-triazin-2-yl)- und N-(4,6-Dimethoxy-s-triazin-2-yl)-N-methyl-O-methyl-urethan, N-methyl-O-ethyl-urethan, -N-methyl-O-benzyl-urethan und -N-methyl-O-phenyl-urethan.

Die Ausgangsstoffe der Formel (III) sind bekannt und/ oder können nach an sich bekannten Verfahren hergestellt werden (vgl. EP-A 101670; US-P 4683000; US-P 4678500; US-P 4786311).

Man erhält die Urethane der Formel (III) nach einem neuen und erfinderischen Verfahren, wenn man Amine der allgemeinen Formel (IV)

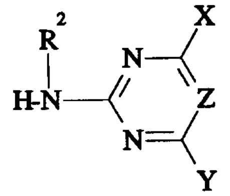

(IV)

in welcher

$R^2$,X, Y und Z die oben angegebene Bedeutung haben,

mit Chlorameisensäureestern der allgemeinen Formel (V)

$R^3$-O-CO-Cl (V)

in welcher

$R^3$ die oben angegebene Bedeutung hat,

gegebenenfalls In Gegenwart eines Säureakzeptors, wie z.B. Natrium(hydrogen)-carbonat, Kalium-(hydrogen)carbonat oder Kalium-tert-butylat, und gegebenenfalls in Gegenwart eines Verdünnungsmittels, wie z.B. Cyclohexan, Methylcyclohexan, Benzol, Toluol, Xylol, Chlorbenzol, Diisopropylether, Dibutylether, Diisobutylether, Methyl-tert-butylether, Tetrahydrofuran, Dioxan, Glykoldimethylether` Aceton, Methylethylketon, Methylisobutylketon, Essigsäure-methylester, -ethylester, -propylester oder -butylester, Acetonitril oder Propionitril, bei Temperaturen zwischen 0 °C und 150 °C umsetzt (vgl. die Herstellungsbeispiele).

Das erfindungsgemäße Verfahren wird unter Einsatz von Alkalimetall-hydroxiden oder -alkoholaten oder von Erdalkalimetall-hydroxiden oder -alkoholaten durchgeführt. Bevorzugt werden Lithium-, Natrium-, Kalium-, Magnesium-und Calcium-hydroxid, Natrium-, Kalium- und Magnesium-methylat und -ethylat, Natrium-und Kalium-propylat, -isopropylat, -butylat, isobutylat, -sec-butylat und -tert-butylat.

Besonders bevorzugt werden Natrium- und Kalium-methylat, Natrium- und Kaliumethylat sowie Natrium- und Kalium-hydroxid.

Die erfindungsgemäße Verfahrensstufe der Herstellung der Sulfonamid-Salze der Formel (IIa) wird vorzugsweise in Gegenwart eines Verdünnungsmittels durchgeführt. Als Verdünnungsmittel sind hierbei insbesondere organische Lösungsmittel geeignet. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutylketon, Ester wie Essigsäuremethylester und -ethylester, Nitrile wie z.B. Acetonitril und Propionitril, Amide wie z.B. Dimethylformamid, Dimethylacetamid und N-Methylpyrrolidon, sowie Alkohole wie Methanol, Ethanol, Propanol, Isopropanol, Butanol, Isobutanol, sec-Butanol und tert-Butanol sowie Wasser. Wasser und Alkohole wie Methanol und Ethanol werden hierbei ganz besonders bevorzugt.

Die Reaktionstemperaturen können bei der erfindungsgemäßen Verfahrensstufe zur Herstellung der Sulfonamid-Salze der Formel (IIa) in einem größeren Bereich variiert werden. Im allgemeinen arbeitet man bei Temperaturen zwischen -20 °C und +150 °C, vorzugsweise zwischen 0 °C und 120 °C.

Die Endstufe des erfindungsgemäßen Verfahrens, d.h. die Umsetzung der Sulfonamid-Salze der Formel (IIa) mit den Urethanen der Formel (III), wird vorzugsweise in Gegenwart eines Verdünnungsmittels durchgeführt. Als Verdünnungsmittel sind hierbei insbesondere aprotische organische Lösungsmittel geeignet. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethyl-ester, Nitrile wie z.B. Acetonitril und Propionitril, Arnide wie z.B. Dimethylformamid, Dimethylacetamid und N-Methyl-pyrrolidon sowie Dimethylsulfoxid, Tetramethylensulfon und Hexamethylphosphorsäuretriamid.

Ketone wie Methyl-isobutylketon und Nitrile wie Acetonitril werden hierbei ganz besonders bevorzugt.

Die Reaktionstemperaturen können bei der Endstufe des erfindungsgemäßen Verfahrens in einem größeren Bereich variiert werden. Im allgemeinen arbeitet man bei Temperaturen zwischen -20 °C und +150 °C, vorzugsweise bei Temperaturen zwischen 0 °C und 100 °C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens setzt man auf 1 Mol Sulfonamid der Formel (II) im allgemeinen zwischen 0,9 und 1,5, vorzugsweise zwischen 0,95 und 1,20 Mol bzw. Moläquivalent eines Alkalimetall-hydroxids oder -alkoholats oder eines Erdalkalimetall-hydroxids oder -alkoholats und zwischen 0,9 und 1,2, vorzugsweise zwischen 0,95 und 1,10 Mol eines Urethans der Formel (III) ein.

In einer bevorzugten Ausführungsform des erfindungsgemäßen Verfahrens wird das Sulfonamid der Formel (11) in einem organischen Verdünnungsmittel vorgelegt und unter Rühren wird das Alkalimetall-hydroxid oder -alkoholat oder das Erdalkalimetallhydroxid oder -alkoholat eindosiert. Das Gemisch wird dann einige Zeit gerührt und dann unter vermindertem Druck eingeengt. Der Rückstand, welcher im wesentlichen das Sulfonamid-Salz der Formel (IIa) enthält wird In einem geeigneten Verdünnungsmittel aufgenommen und dann unter Rühren mit dem Urethan der Formel (III) versetzt.

In einer weiteren bevorzugten Ausführungsform des erfindungsgemäßen Verfahrens wird das Sulfonainid der Formel (II) mit der wäßrigen Lösung der äquivalenten Menge eines Alkalimetall- oder Erdalkalimetall-hydroxids bis zur praktisch vollständigen Auflösung verrührt. Dann wird gegebenenfalls von ungelösten Bestandteilen abfiltriert und zur Lösung ein organisches Lösungsmittel, vorzugsweise Methyl-isobutylketon oder Toluol, gegeben. Das Wasser wird dann durch azeotrope Destillation entfernt. Zur verbleibenden Lösung wird dann, vorzugsweise bei Temperaturen zwischen 60 °C und 90 °C, das Urethan der Formel (III) gegeben und die Reaktionsmischung wird bis zum Ende der Umsetzung gerührt.

Nach dem Ende der Umsetzung kann auf übliche Weise aufgearbeitet werden (vgl. die Herstellungsbeispiele).

Die nach dem erfindungsgemäßen Verfahren herzustellenden Sulfonylharnstoff-Salze der Formel I) können als selektive Herbizide eingesetzt werden (vgl. EP-A 251 079 und EP-A 433 779).

Herstellungsbeispiele:

Beispiel 1

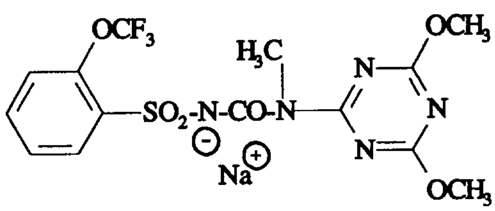

12,1 g (50,2 mMol) 2-Trifluormethoxy-benzolsulfonamid werden in 50 ml Methanol aufgenommen und bei 20 °C unter Rühren mit 9,1 g einer 30%igen Lösung von Natrium-methylat in Methanol (50,6 mMol $NaOCH_3$) versetzt. Das Gemisch wird dann noch 2 Stunden bei 20 °C gerührt und anschließend im Wasserstrahlvakuum eingeengt. Der Rückstand wird In Toluol aufgenommen und erneut eingeengt. Dann wird der Rückstand in 100 ml Acetonitrll aufgenommen und unter Rühren bei 20 °C mit 14,5 g (45,8 mMol)

N-Methyl-N-(4,6-dimethoxy-1,3,5-triazin-2-yl)-carbamidsäure-phenylester versetzt. Nach einiger Zeit entsteht aus der Suspension eine hellgelbe, fast klare Lösung, aus der allmählich ein weißer Feststoff ausfällt. Nach 12 Stunden wird das Produkt durch Absaugen isoliert.

Man erhält 19,9 g (93% der Theorie) 3-(4,6-Dimethoxy-s-triazin-2-yl)-3-methyl-1-(2-trifluormethoxyphenylsulfonyl)-harnstoff-Natriumsalz vom Schmelzpunkt 224 °C (Gehalt nach HPLC: 98,3%).

Analog Beispiel 1 und entsprechend der allgemeinen Beschreibung des erfindungsgemäßen Verfahrens können beispielsweise auch die in der nachstehenden Tabelle 1 aufgeführten Verbindungen der Formel (1) hergestellt werden.

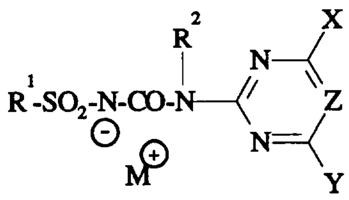

(I)

**Tabelle 1:** Beispiele für die erfindungsgemäß herzustellenden Verbindungen der Formel (I)

| Bsp.-Nr. | $M^+$ | $R^1$ | $R^2$ | X | Y | Z | Schmp. [°C] | Ausbeute (% der Theorie) |
|---|---|---|---|---|---|---|---|---|
| 2 | $Na^+$ | 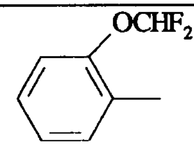 | $CH_3$ | $OCH_3$ | $OCH_3$ | N | 201 | 94 |
| 3 | $Na^+$ | 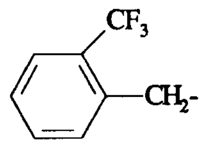 | $CH_3$ | $OCH_3$ | $OCH_3$ | N | >230 | 95 |
| 4 | $Na^+$ | 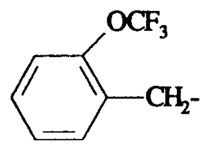 | $CH_3$ | $OCH_3$ | $OCH_3$ | N | 188 | 88 |
| 5 | $Na^+$ | 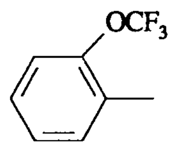 | $CH_3$ | $CH_3$ | $OCH_3$ | N | 194 | 87 |
| 6 | $Na^+$ | 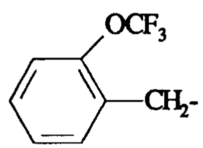 | $CH_3$ | $CH_3$ | $OCH_3$ | N | 200 | 88 |

**Tabelle 1** - Fortsetzung

| Bsp.-Nr. | $M^+$ | $R^1$ | $R^2$ | X | Y | Z | Schmp. [°C] | Ausbeute (% der Theorie) |
|---|---|---|---|---|---|---|---|---|
| 7 | $Na^+$ | 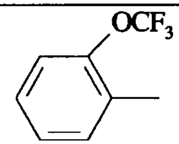 | H | $OCH_3$ | $OCH_3$ | CH | 190 | 59 |
| 8 | $Na^+$ | 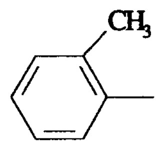 | $CH_3$ | $OCH_3$ | $OCH_3$ | N | 135 | 76 |
| 9 | $Na^+$ | 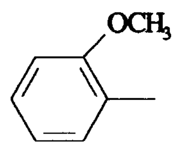 | $CH_3$ | $OCH_3$ | $OCH_3$ | N | 165 | 78 |
| 10 | $Na^+$ | 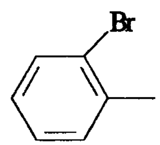 | $CH_3$ | $OCH_3$ | $OCH_3$ | N | 195 | 90 |
| 11 | $Na^+$ | 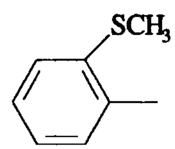 | $CH_3$ | $OCH_3$ | $OCH_3$ | N | 130 | 91 |

**Tabelle 1** - Fortsetzung

| Bsp.-Nr. | $M^+$ | $R^1$ | $R^2$ | X | Y | Z | Schmp. [°C] | Ausbeute (% der Theorie) |
|---|---|---|---|---|---|---|---|---|
| 12 | $Na^+$ | 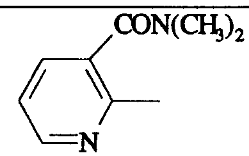 | $CH_3$ | $OCH_3$ | $OCH_3$ | N | 220 | 75 |
| 13 | $Na^+$ | 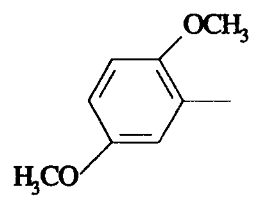 | $CH_3$ | $OCH_3$ | $OCH_3$ | N | 225 | 88 |

Die gemäß Beispiel 1 erhaltene Verbindung kann beispielsweise auch wie folgt hergestellt werden:

Beispiel 1a

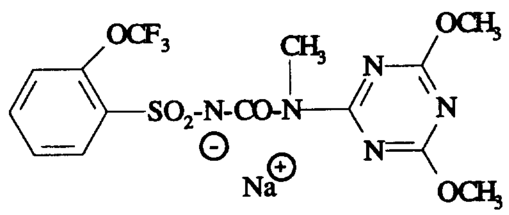

14,4 g (60 mMol) 2-Trifluormethoxy-benzolsulfonamid werden mit einer Lösung von 2,4 g (60 mMol) Natriumhydroxid in 50 ml Wasser bis zur praktisch vollständigen Auflösung bei 20 °C gerührt. Nach Filtration wird die klare Lösung mit 150 ml Methyl-isobutylketon versetzt und das Wasser durch Azeotropdestillation entfernt. Anschließend werden zur verbleibenden Lösung bei 70 °C bis 90 °C 18,8 g (60,6 mMol) N-Methyl-N-(4,6-dimethoxy-1,3,5-triazin-2-yl)-carbamidsäure-phenylester gegeben. Es bildet sich eine klare Lösung, aus der nach kurzer Zeit das Produkt voluminös ausfällt. Zwecks besserer Rührbarkeit wird auf 500 ml (mit Methylisobutylketon) verdünnt und die Mischung wird 12 Stunden bei 20 °C gerührt. Das Produkt wird dann durch Absaugen isoliert.

Man erhält 24 g (84% der Theorie) 3-(4,6-Dimethoxy-s-triazin-2-yl)-3-methyl-1-(2-trifluormethoxy-phenylsulfonyl)-harnstoff-Natriumsalz vom Schmelzpunkt 224 °C (Gehalt nach HPLC: 96,7%).

Die in Tabelle 1 als Beispiel 2 aufgeführte Verbindung kann beispielsweise wie folgt hergestellt werden:

Beispiel 2a

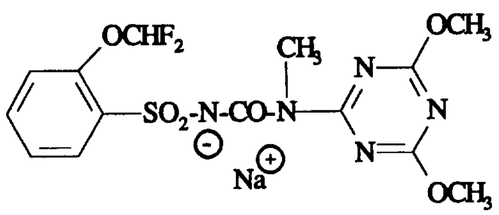

6,7 g (30 mMol) 2-Difluormethoxy-benzolsulfonamid werden mit einer Lösung von 1,2 g (30 mMol) Natriumhydroxid in 30 ml Wasser bis zur praktisch vollständigen Auflösung gerührt. Nach Filtration wird die klare Lösung mit 300 ml Methylisobutylketon versetzt und das Wasser wird durch Azeotropdestillation entfernt. Anschließend werden zur verbleibenden Mischung bei 70 °C bis 80 °C 9,3 g (30 mMol) N-Methyl-N-(4,6-dimethoxy-1,3,5-triazin-2-yl)-carbamid-säure-phenylester gegeben. Das Reaktionsgemisch wird noch 12 Stunden bei 20 °C gerührt; dann wird das kristallin angefallene Produkt durch Absaugen isoliert.

Man erhält 11,4 g (77% der Theorie) 3-(4,6-Dimethoxy-s-triazin-2-yl)-3-methyl-1-(2-difluormethoxy-phenylsulfonyl)-harnstoff-Natriumsalz vom Schmelzpunkt 201 °C (Gehalt nach HPLC: 88,9%).

Ausgangsstoffe der Formel (III):

Beispiel (III)-1)

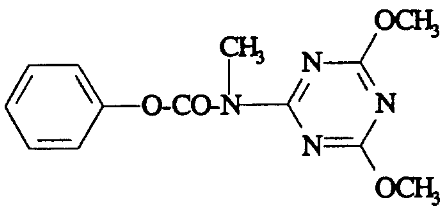

Zu 5,0g (0,0294 Mol) 2-Methylamino-4,6-dimethoxy-1,3,5-triazin in 100 ml Tetrahydrofuran werden 3,7 g (0,033 Mol) K-t-butylat gegeben. Unter gutem Rühren werden 5,1 g (0,0326 Mol) Chlorameisensäure-phenylester schnell zugetropft, wobei der Reaktionsansatz durch Kühlung auf ca 25 °C gehalten wird. Nach 12h bei 20 °C wird im Vakuum eingeengt, in Methylenchlorid aufgenommen und mit Wasser neutral gewaschen. Nach Trocknen mit Magnesiumsulfat wird eingeengt, der Rückstand mit Ether/Petrolether (1:1) verrührt. Das kristallin ausgefallene Produkt wird abgesaugt und getrocknet. Ausbeute: 6,6 g (73% der Theorie) N-Methyl-N-(4,6-dimethoxy-1,3,5-triazin-2-yl)-carbamidsäure-phenylester vom Schmp. 90 °C.

Beispiel (III-2)

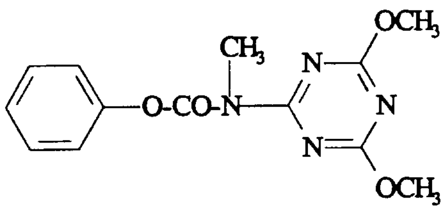

Zu einer Mischung aus 5,0 g (0,0294 Mol) 2-Methylamino-4,6-dimethoxy-1,3,5-triazin und 5,5 g (0,0655 Mol) Natriumhydrogencarbonat in 100 ml Cyclohexan werden innerhalb von 1,5 Stunden unter Rühren 10,2 g (0,0651 Mol) Chlorameisensäure-phenylester bei 80 °C zugetropft. Es wird 12h unter Rückfluß am Wasserabscheider erhitzt, abgekühlt und eingeengt. Der Rückstand wird in Methylenchlorid aufgenommen und mit Wasser neutral gewaschen, getrocknet und eingeengt. Nach Verrühren mit Ether/Petrolether 1:1 werden 5,3 g weiße Kristalle (59% der Theorie) N-Methyl-N-(4,6-dimethoxy-1,3,5-triazin-2-yl)-carbamidsäure-phenylester vom Schmp. 90 °C erhalten.

Analog können beispielsweise auch die in der nachstehenden Tabelle 2 aufgeführten Verbindungen der Formel (III) hergestellt werden.

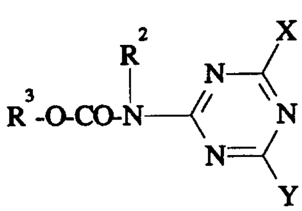

(III)

Tabelle 2

| Beispiele für die Verbindungen der Formel (III) | | | | | | |
|---|---|---|---|---|---|---|
| Bsp.-Nr. | $R^2$ | $R^3$ | X | Y | Z | Schmelzpunkt (°C) |
| III-3 | $CH_3$ | $C_6H_5$ | $CH_3$ | $OCH_3$ | N | 82 |
| III-4 | $CH_3$ | $C_6H_5$-$CH_2$ | $OCH_3$ | $OCH_3$ | N | 77 |
| III-5 | $CH_3$ | $C_2H_5$ | $OCH_3$ | $OCH_3$ | N | 45 |
| III-6 | $CH_3$ | $CH_3$ | $OCH_3$ | $OCH_3$ | N | 69 |
| III-7 | $C_2H_5$ | $C_6H_5$ | $CH_3$ | $CH_3$ | N | |
| III-8 | $C_2H_5$ | $C_6H_5$ | $CH_3$ | $OCH_3$ | N | |
| III-9 | $C_2H_5$ | $C_6H_5$ | $OCH_3$ | $OCH_3$ | N | 61-62 |
| III-10 | $C_3H_7$-n | $C_6H_5$ | $OCH_3$ | $OCH_3$ | N | |
| III-11 | $C_3H_7$-i | $C_6H_5$ | $OCH_3$ | $OCH_3$ | N | |
| III-12 | $CH_2$-CH = $CH_2$ | $C_6H_5$ | $OCH_3$ | $OCH_3$ | N | |
| III-13 | $C_3H_7$-n | $C_6H_5$ | $CH_3$ | $OCH_3$ | N | |

**Tabelle 2** - Fortsetzung

| Bsp.-Nr. | $R^2$ | $R^3$ | X | Y | Z | Schmelzpunkt (°C) |
|---|---|---|---|---|---|---|
| III-14 | $C_3H_7$-i | $C_6H_5$ | $CH_3$ | $OCH_3$ | N | |
| III-15 | $CH_2$-C≡CH | $C_6H_5$ | $OCH_3$ | $OCH_3$ | N | |
| III-16 | $CH_2$-C≡CH | $C_6H_5$ | $CH_3$ | $OCH_3$ | N | |
| III-17 | $CH_2$-$C_6H_5$ | $C_6H_5$ | $OCH_3$ | $OCH_3$ | N | |

Die gemäß Beispiel (III-2) erhaltene Verbindung kann beispielsweise auch wie folgt hergestellt werden:

Beispiel (III-.2a)

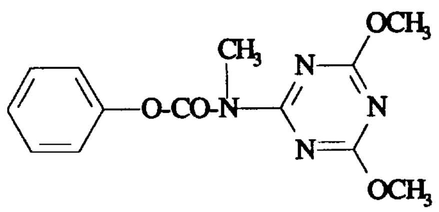

Eine Mischung aus 89,6 g (0,52 Mol) 2-Methylamino-4,6-dimethoxy-1,3,5-triazin, 84 g (1,0 Mol) Natriumhydrogencarbonat und 800 ml Essigsäureburylester wird am Wasserabscheider unter Rückfluß erhitzt und im Verlauf von 8 Stunden wird eine Lösung von 102 g (0,65 Mol) Chlorameisensäurephenylester in 500 ml Essigsäurebutylester zugetropft. Nach einer weiteren Stunde unter Rückfluß läßt man abkühlen und wäscht die Reaktionslösung mit Wasser. Das Waschwasser wird mit 2N-Salzsäure neutralisiert und mit Essigsäurebutylester nachextrahiert. Die vereinigten organischen Lösungen werden mit Natriumsulfat getrocknet und filtriert. Das Filtrat wird eingeengt, der ölige Rückstand in Methylcyclohexan aufgenommen und intensiv gerührt. Das hierbei kristallin angefallene Produkt wird durch Absaugen isotiert.

Man erhält 132 g (80% der Theorie) N-Methyl-N-(4,6-dimethoxy-1,3,5-triazin-2-yl)-carbarnidsäure-phenylester vom Schmelzpunkt 90 ° C.

Beispiel (III-2b)

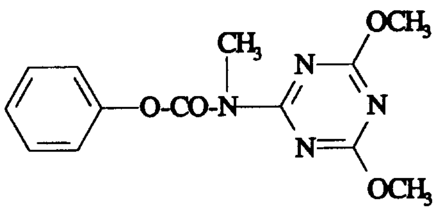

Eine Mischung aus 17 g (0,1 Mol) 2-Methylamino-4,6-dimethoxy-1,3,5-triazin, 12,5 g (0,15 Mol) Natriumhydrogencarbonat und 125 ml Methylisobutylketon wird am Wasserabscheider unter Rückfluß erhitzt und im Verlauf von 2,5 Stunden wird eine Losung von 23,5 g (0,15 Mol) Chlorameisensäurephenylester in 25 ml Methylisobutylketon zugetropft. Nach weiteren 2,5 Stunden unter Rückfluß wird auf 30 ° C abgekühlt und mit 1%iger Sodalösung gewaschen. Die wäßrige Phase wird mit Methylisobutylketon nachextrahiert. Die vereinigten organischen Lösungen werden mit Natriumsulfat getrocknet und filtriert. Das Filtrat wird eingeengt und der erhaltene Rückstand aus Cyclohexan umkristallisiert.

Man erhält 21,8 g (72% der Theorie) N-Methyl-N-(4,6-dimethoxy-1,3,5-triazin-2-yl)-carbamidsäure-phenylester vom Schmelzpunkt 90 ° C.

Beispiel (III-2c)

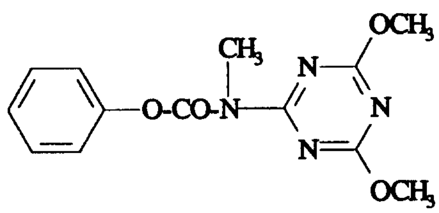

172 g (1,0 Mol) 2-Methylamino-4,6-dimethoxy-1,3,5-triazin und 168 g (2,0 Mol) Natriumhydrogencarbonat werden in 1 Liter Toluol am Wasserabscheider unter Rückfluß erhitzt. Im Verlauf von 5 Stunden werden 235 g (1,5 Mol) Chlorameisensäurephenylester tropfenweise dazugegeben und das komplette Reaktionsgemisch wird dann noch eine Stunde lang unter Rückfluß gerühr. Nach Abkühlen auf 40 ° C wird filtriert und vom Filtrat wird das Lösungsmittel im Vakuum sorgfältig abdestilliert.

Man erhält 348 g (91% der Theorie) N-Methyl-N-(4,6-dimethoxy-1,3,5-triazin-2-yl)-carbamidsäure-phenylester als gelblichen Feststoff.

## Patentansprüche

**1.** Verfahren zur Herstellung von Sulfonylharnstoff-Salzen der allgemeinen Formel (I)

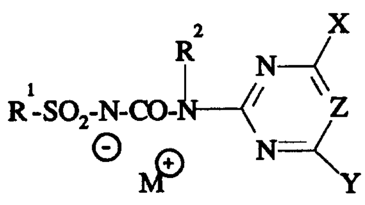

(I)

in welcher

$M^+$ für ein Alkalimetallion oder ein Erdalkalimetallionenäquivalent steht,
$R^1$ für jeweils gegebenenfalls substituiertes Aryl, Aralkyl oder Heteroaryl steht,
$R^2$ für Wasserstoff oder für jeweils gegebenenfalls substituiertes Alkyl, Alkenyl, Alkinyl oder Aralkyl steht,
X für Wasserstoff, Halogen oder für jeweils gegebenenfalls durch Halogen oder Alkoxy substituiertes Alkyl, Alkoxy, Alkylthio oder Alkylamino steht,
Y für Wasserstoff, Halogen oder für jeweils gegebenenfalls durch Halogen substituiertes Alkyl oder Alkoxy steht und
Z für Stickstoff, eine CH-Gruppierung oder eine C-Halogen-Gruppierung steht,

dadurch gekennzeichnet, daß man zunächst Sulfonsäureamide der allgemeinen Formel (II)

$R^1$-$SO_2$-$NH_2$ (II)

in welcher
$R^1$ die oben angegebene Bedeutung hat,
mit Alkalimetall-hydroxiden oder -alkoholaten oder mit Erdalkalimetallhydroxiden oder -alkoholaten gegebenenfalls in Gegenwart eines Verdünnungsmittels bei Temperaturen zwischen -20 °C und +150 °C umsetzt (1. Stufe)
und dann die hierbei gebildeten Sulfonamid-Salze der allgemeinen Formel (IIa)

$R^1$-$SO_2$-NH-$M^+$ (IIa)

in welcher
M und $R^1$ die oben angegebene Bedeutung haben,
mit Urethanen (Carbamaten) der allgemeinen Formel (III)

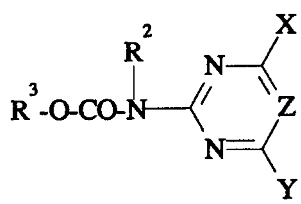
(III)

in welcher
$R^2$, X, Y und Z die oben angegebene Bedeutung haben und
$R^3$ für Alkyl, Aralkyl oder Aryl steht,
gegebenenfalls in Gegenwart eines Verdünnungsmittels bei Temperaturen zwischen -20 °C und +150 °C umsetzt (2. Stufe) und die Produkte der Formel (I) nach üblichen Methoden isoliert.

2. Verfahren gemäß Anspruch 1 zur Herstellung von Verbindungen der Formel (I), dadurch gekennzeichnet, daß in Formel (I)
$M^+$ für ein Lithium-, Natrium- oder Kaliumion oder für ein Magnesium- oder Calciumionenäquivalent steht,
$R^1$ für jeweils gegebenenfalls substituiertes Phenyl, Naphthyl, Benzyl, Pyridyl, Thienyl oder Parazolyl steht, wobei die möglichen Substituenten vorzugsweise ausgewählt sind aus der Reihe Halogen, Carboxy, Cyano, Carbamoyl, Nitro, Amino, $C_1$-$C_4$-Alkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkoxy-$C_1$-$C_4$-alkoxy, $C_1$-$C_4$-Alkylthio, $C_1$-$C_4$-Alkylsulfinyl, $C_1$-$C_4$-Alkylsulfonyl, Di-($C_1$-$C_4$-alkyl)-amino-sulfonyl, N-$C_1$-$C_4$-Alkoxy-N-$C_1$-$C_4$-alkyl-amino-sulfonyl, Phenyl, Phenoxy, $C_1$-$C_4$-Alkoxy-carbonyl, $C_1$-$C_4$-Halogenalkoxy-carbonyl, $C_1$-$C_2$-Alkoxy-$C_1$-$C_2$-alkoxy-carbonyl, Di-($C_1$-$C_4$-alkyl)-amino-carbonyl,
$R^2$ für Wasserstoff, für $C_1$-$C_6$-Alkyl (welches gegebenenfalls durch Fluor, Cyano, $C_1$-$C_4$-Alkoxy oder $C_1$-$C_4$-Alkylthio substituiert ist), für $C_3$-$C_6$-Alkenyl und $C_3$-$C_6$-Alkinyl (welche gegebenenfalls durch Fluor oder Chlor substituiert sind) oder für Phenyl-$C_1$-$C_2$-alkyl (welches im Phenylteil gegebenenfalls durch Fluor, Chlor, Nitro, Cyano, Methyl, Methoxy oder $C_1$-$C_2$-Alkoxycarbonyl substituiert ist) steht,
X für Wasserstoff, Halogen oder für jeweils gegebenenfalls durch Halogen oder $C_1$-$C_4$-Alkoxy

substituiertes $C_1$-$C_4$-Alkyl, $C_1$-$C_4$-Alkoxy, $C_1$-$C_4$-Alkylthio oder $C_1$-$C_4$-Alkylamino steht,

Y für Wasserstoff, Halogen oder für jeweils gegebenenfalls durch Halogen substituiertes $C_1$-$C_4$-Alkyl oder $C_1$-$C_4$-Alkoxy steht und

Z für Stickstoff oder eine CH-Gruppierung steht.

**3.** Verfahren nach Anspruch 1 zur Herstellung von Verbindungen der Formel (I), dadurch gekennzeichnet, daß in Formel (I)

$M^+$ für ein Nathumion oder ein Kaliumion steht,

$R^1$ für jeweils in ortho-Position durch Fluor, Chlor,Brom, Methyl, Ethyl, Trifluormethyl, Methoxy, Ethoxy, Difluormethoxy, Trifluormethoxy, Chlorethoxy, Methoxyethoxy, Ethoxyethoxy, Methylthio, Ethylthio, Propylthio, Methylsulfinyl, Ethylsulfinyl, Propylsulfinyl, Methylsulfonyl, Ethylsulfonyl, Propylsulfonyl, Dimethylaminosulfonyl, Diethylarninosulfonyl, N-Methoxy-N-methylaminosulfonyl, Phenyl, Methoxycarbonyl, Ethoxycarbonyl, Propoxycarbonyl, Isoprop-oxycarbonyl, Chlorethoxycarbonyl, Methoxyethoxycarbonyl, Dimethylarninocarbonyl oder Diethylaminocarbonyl und gegebenenfalls in einer weiteren Position durch Halogen substituiertes Phenyl oder Benzyl, für 3-Dimethylaminocarbonylpyridin-2-yl oder 3-Ethylsulfonyl-pyridin-2-yl, für 2-Methoxycarbonylthiophen-3-yl oder für 1-Methyl-4-methoxycarbonyl-pyrazol-5-yl, 1-Methyl-4-ethoxycarbonyl-pyrazol-5-yl, 1-Methyl-3-chlor-4-methoxy-carbonyl-pyrazol-5-yl, 1-Methyl-3-chlor-4-ethoxycarbonyl-pyrazol-5-yl oder 1-Pyridyl-4-methoxycarbonyl-pyrazol-5-yl steht,

$R^2$ für Wasserstoff oder Methyl steht,

X für Wasserstoff, Chlor, Methyl, Ethyl, Trichlormethyl, Trifluormethyl, Methoxymethyl, Methoxy, Ethoxy, Difluormethoxy, Fluorethoxy, Difluorethoxy, Trifluorethoxy, Methoxyethoxy, Methylthio, Ethylthio, Methylamino oder Ethylamino steht,

Y für Wasserstoff, Chlor, Methyl, Ethyl, Trifluormethyl, Methoxy, Ethoxy oder Difluormethoxy steht, und

Z für Stickstoff oder eine CH-Gruppierung steht.

**4.** Verfahren nach Anspruch 1, dadurch gekennzeichnet, daß die 1. Verfahrensstufe bei Temperaturen zwischen 0 °C und 120 °C durchgeführt wird.

**5.** Verfahren nach Anspruch 1, dadurch gekennzeichnet, daß die 2. Verfahrensstufe bei Temperaturen zwischen 0 °C und 100 °C durchgeführt wird.

**6.** Verfahren nach Anspruch 1, dadurch gekennzeichnet, daß man in der 1. Verfahrensstufe als Verdünnungsmittel Wasser oder einen Alkohol einsetzt, vorzugsweise Methanol und Ethanol.

**7.** Verfahren nach Anspruch 1, dadurch gekennzeichent, daß man in der 2. Verfahrensstufe als Verdünnungsmittel ein Keton oder ein Nitril einsetzt, vorzugsweise Methyl-isobutylketon oder Acetonitril.

**8.** Verfahren nach Anspruch 1, dadurch gekennzeichnet, daß man das Sulfonamid der Formel (II) in einem organischen Verdünnungsmittel vorlegt und unter Rühren das Alkalimetall-hydroxid oder -alkoholat oder das Erdalkalimetallhydroxid oder -alkoholat eindosiert, das Gemisch dann einige Zeit rührt und dann unter vermindertem Druck einengt, schließlich den Rückstand - der im wesentlichen das Sulfonamid-Salz der Formel (IIa) enthält - in einem geeigneten Verdünnungsmittel aufnimmt und dann unter Rühren mit dem Urethan der Formel (III) versetzt.

**9.** Verfahren zur Herstellung von Urethanen der Formel (III),

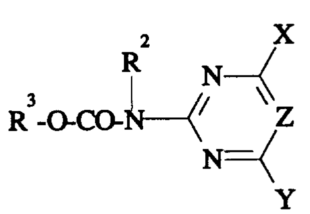

(III)

in welcher

$R^2$ für Wasserstoff oder für jeweils gegebenenfalls substituiertes Alkyl, Alkenyl, Alkinyl oder Aralkyl steht,

X für Wasserstoff, Halogen oder für jeweils gegebenenfalls durch Halogen oder Alkoxy substituiertes Alkyl, Alkoxy, Alkylthio oder Alkylamino steht,

Y für Wasserstoff, Halogen oder für jeweils gegebenenfalls durch Hälogen substituiertes Alkyl oder Alkoxy steht,

Z für Stickstoff, eine CH-Gruppierung oder eine C-Halogen-Gruppierung steht, und

$R^3$ für Alkyl, Aralkyl oder Aryl steht,

dadurch gekennzeichnet, daß man Amine der allgemeinen Formel (IV)

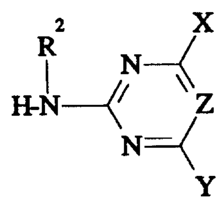

(IV)

in welcher

$R^2$, X, Y und Z die oben angegebene Bedeutung haben,

mit Chlorameisensäureestern der allgemeinen Formel (V)

$R^3$-O-CO-Cl (V)

in welcher

$R^3$ die oben angegebene Bedeutung hat,

gegebenenfalls in Gegenwart eines Säureakzeptors [vorzugsweise Natrium(hydrogen)carbonat, Kalium-(hydrogen)carbonat oder Kalium-tert-butylat], und gegebenenfalls in Gegenwart eines Verdünnungsmittels [vorzugsweise Cyclohexan, Methylcyclohexan, Benzol, Toluol, Xylol, Chlorbenzol, Diisopropylether, Dibutylether, Diisobutylether, Methyl-tert-butylether, Tetrahydrofuran, Dioxan, Glykoldimethylether, Aceton, Methylethylketon, Methylisobutylketon, Essigsäure-methylester, -ethylester, -propylester oder -butylester, Acetonitril oder Propionitril], bei Temperaturen zwischen 0 °C und 150 °C umsetzt.

Europäisches Patentamt

# EUROPÄISCHER RECHERCHENBERICHT

Nummer der Anmeldung

EP 93 10 2758

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | EP-A-0 433 779 (BAYER AG)<br>--- | 1-9 | C07D251/46<br>A01N47/36 |
| A,D | EP-A-0 304 282 (E.I. DU PONT DE NEMOURS AND COMPANY)<br>--- | 1-9 | C07D251/16<br>C07D239/52 |
| A,D | EP-A-0 101 670 (CIBA-GEIGY AG)<br>--- | | |
| A | DE-A-3 609 700 (SCHERING AKTIENGESELLSCHAFT)<br>----- | | |

RECHERCHIERTE SACHGEBIETE (Int. Cl.5 )

C07D
A01N

Der vorliegende Recherchenbericht wurde für alle Patentansprüche erstellt

| Recherchenort | Abschlußdatum der Recherche | Prüfer |
|---|---|---|
| DEN HAAG | 11 JUNI 1993 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)